# EUROPÄISCHE PATENTSCHRIFT

(11) **EP 0 667 341 B1**
(45) Veröffentlichungstag und Bekanntmachung des Hinweises auf die Patenterteilung: **29.08.2001**
(21) Anmeldenummer: 95101575.9
(22) Anmeldetag: 07.02.1995
(51) Int. Cl.: C07D 211/58, A61K 31/445, C07D 401/04, C07D 401/12

(54) **4-Amino-1-piperidylbenzoylguanidine als Na+/H+-antiporter Inhibitoren**
4-Amino-1-piperidylbenzoylguanidines as Na+/H+ antiporter inhibitors
4-Amino-1-pipéridylbenzoyl guanidines comme inhibiteurs Na+/H+ antiporter

(30) Priorität: 10.02.1994 DE 4404183
(43) Veröffentlichungstag der Anmeldung: 16.08.1995
(73) Patentinhaber: MERCK PATENT GmbH, 64293 Darmstadt (DE)
(72) Erfinder: Gericke, Rolf, Dr., D-64342 Seeheim-Jugenheim (DE); Baumgarth, Manfred, Dr., D-64372 Darmstadt (DE); Dorsch, Dieter, Dr., D-64372 Ober-Ramstadt (DE); Beier, Norbert, Dr., D-64354 Reinheim (DE); Minck, Klaus-Otto, Dr., D-64372 Ober-Ramstadt (DE); Lues, Ingeborg, Dr., D-64297 Darmstadt (DE)

(56) Entgegenhaltungen:
- EP-A- 0 416 499
- EP-A- 0 556 672
- EP-A- 0 589 336
- EP-A- 0 600 371

## Beschreibung

Die Erfindung betrifft N-Diaminomethylen-4-(4-amino-piperidino)-5-methylsulfonyl-benzamid-derivate ausgewählt aus der Gruppe
a) N-Diaminomethylen-4-(4-amino-piperidino)-2-methyl-5-methylsulfonyl-benzamid,
b) N-Diaminomethylen-4-(4-amino-piperidino)-2-methoxy-5-methylsulfonyl-benzamid,
c) N-Diaminomethylen-4-(4-amino-piperidino)-2-chlor-5-methylsulfonylbenzamid,
sowie deren physiologisch unbedenklichen Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1982)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 04 16 499.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na+/H+-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. counillon, W. Scholz, H.L. Land and J. Pouysségur in Mol. Pharmacol. 44, 1041-1045 (1993) angegeben werden.

Als Versuchstiere eignen sich z.B. Mäuse, Ratten, Meerschweinchen, Hunde, Katzen, Affen oder Schweine.

Die Verbindungen können daher als Arzneimittelwirkstoffe in der Human-und Veterinärmedizin verwendet werden. Ferner können sie als Zwischenprodukte zur Herstellung weiterer Arzneimittelwirkstoffe verwendet werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Ihre Art und Größe ist im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitet Acylgruppen sowie insbesondere Alkoxycarbonyl-, Aryloxycarbonyl-und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA (Phenoxyacetyl); Alkoxycarbonyl wie Methoxycarbonyl, Ethoxycarbonyl, 2,2,2-Trichlorethoxycarbonyl, BOC (tert.-Butoxycarbonyl), 2-Jodethoxycarbonyl; Aralkyloxycarbonyl wie CBZ ("Carbobenzoxy"), 4-Methoxybenzyloxycarbonyl, FMOC (9-Fluorenylmethoxycarbonyl); Arylsulfonyl wie Mtr (4-Methoxy-2,3,6-trimethylphenyl-sulfonyl). Bevorzugte Aminoschutzgruppen sind BOC, ferner CBZ, FMOC, Benzyl und Acetyl.

Die erfindungsgemäßen Verbindungen werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die erfindungsgemäßen Verbindungen hergestellt, indem man ein aktiviertes Carbonsäurederivat mit Guanidin umsetzt. Besonders geeignet sind Reaktionsvarianten, bei denen die freie Carbonsäure in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukayama-Variante (Angew. Chem. 91, 788-812 (1979), wobei letztere besonders geeignet ist.

Die Carbonsäuren werden durch nucleophile aromatische Substitution ausgehend von geeigneten Benzoesäurederivaten durch Umsetzung mit entsprechenden p-substituierten Piperidinen hergestellt. Die Umsetzung erfolgt in Analogie zu der wie nachstehend beschrieben.

Die Umsetzung eines reaktionsfähigen Carbonsäurederivates mit Guanidin erfolgt in an sich bekannter Weise vorzugsweise in einem protischen oder aprotischen polaren oder unpolaren inerten organischen Lösungsmittel.

Geeignete Lösungsmittel werden nachfolgend für die Umsetzung genannt. Besonders bevorzugte Solvention sind jedoch Methanol, THF, Dimethoxyethan, Dioxan oder daraus herstellbare Gemische sowie Wasser. Als Reaktionstemperatur sind Temperaturen zwischen 20° und dem Siedepunkt des Lösungsmittels geeignet. Die Reaktionszeiten liegen zwischen 5 Min. und 12 Std.. Es ist zweckmäßig bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Verbindungen gemäß Anspruch 1 können ferner hergestellt werden, indem man ein Benzoylguanidin mit einem Piperidin umsetzt. Die Ausgangsstoffe können auf einfache Weise durch Umsetzung von entsprechend substituierten Benzoesäuren oder daraus ableitbaren reaktionsfähigen Säurederivaten, wie z.B. Säurehalogeniden, Estern oder Anhydriden, mit Guanidin, unter Reaktionsbedingungen wie sie für die Amidherstellung an sich bekannt und allgemein üblich sind, hergestellt werden.

Die Piperidine sind ebenso wie die Methoden zu ihrer Herstellung an sich bekannt. Sofern sie nicht bekannt sind, können sie nach den an sich bekannten Methoden hergestellt werden. So kann beispielsweise ein 4-Aminopyridin zum entsprechenden 4-Aminopiperidin hydriert und dann gegebenenfalls weiter zu den verschiedenen N-substituierten 4-Aminopiperidinen umgesetzt werden.

Bevorzugte Reaktionen sind hierbei Alkylierungen und Acylierungen, insbesondere auch die Einführung von Schutzgruppen.

Desweiteren ist es möglich, daß man in einem 4-Halogenpiperidin das Halogenatom ersetzt. Besonders bevorzugt sind Reaktionen mit cyclischen Aminen wie Pyrrolidin oder primären oder sekundären aromatischen Aminen, wie z.B. Anilin oder daraus ableitbaren Derivaten.

Bei den genannten Herstellungen ist es zweckmäßig, in Gegenwart einer Base oder mit einem Überschuß der basischen Komponente zu arbeiten. Als Basen eignen sich bevorzugt z.B. Alkalimetall- oder Erdalkalimetallhydroxide, -carbonate, -alkoholate oder organische Basen wie Triethylamin oder Pyridin, die auch im Überschuß angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Eine besonders bevorzugte Arbeitsweise besteht darin, daß man die Substanzen direkt, ohne Zusatz von Lösungsmitteln bei Temperaturen zwischen 100° und 400°, besonders bevorzugt bei 100° bis 200° zusammenschmilzt.

Derivate mit primärer oder sekundärer Aminogruppe werden zweckmäßig in geschützter Form umgesetzt, unabhängig davon, ob die Reaktion in Gegenwart eines Lösungsmittels oder in der Schmelze ausgeführt wird.

Als Schutzgruppen kommen die üblichen Aminoschutzgruppen in Frage, wie sie z.B. in der Peptidchemie verwendet werden. Einige charakteristische Gruppen sind beispielsweise 2-Alkoxy-ethoxy-methyl wie 2-Methoxyethoxy-methyl ("MEM"; abspaltbar z.B. mit ZnBr₂ oder TiCl₄ in Dichlormethan) oder 2-Trialkylsilyl-ethoxy-methyl wie 2-Trimethylsilyl-ethoxymethyl ("SEM"; abspaltbar z.B. mit F-lonen). Besonders bevorzugt ist allerdings tert.-Butoxy-carbonyl ("BOC"; abspaltbar mit H⁺).

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z.B. Verbindungen der Formeln A-CI, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z.B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungsmittel, z.B. DMF, bei Temperaturen zwischen etwa 0° und etwa 120° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z.B. Chloride oder Bromide) oder Anhydride von Carbonsäuren der Formel Ac-OH, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0° und etwa 160°, vorzugsweise zwischen 20° und 120°. Eine Formylierung gelingt auch mit Ameisensäure in Gegenwart von Pyridin.

Eine Base einer erfindungsgemäßen Verbindung kann mit einer Säure in das zugehörige Säureadditionssalz übergeführt werden. Für diese Umsetzung kommen Säuren in Frage, die physiologische unbedenkliche Salze liefern. So können anorganische Säure verwendet werden, z.B. Schwefelsäure. Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure. Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure.

Die erfindungsgemäßen Verbindungen und ihre physiologisch unbedenklichen Salze könne zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine erfindungsgemäße Verbindung und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die erfindungsgemäßen Verbindungen und ihre physiologisch unbedenklichen Salze können an Menschen oder Tiere, insbesondere Säugetiere wie Affen, Hunde, Katzen, Ratten oder Mäuse verabreicht und bei der thera-peutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Man rührt eine Lösung von 550 mg 3-Methylsulfonyl-4-(4-BOC-aminopiperidino)-benzoesäuremethylester (F. 149-150°) [erhältlich durch Umsetzung von 3-Methylsulfonyl-4-chlor-benzoesäure mit 4-BOC-aminopiperidin in der Schmelze und anschließende Veresterung des Produkts mit Methyliodid/K₂CO₃ in Dimethylformamid (DMF)] und 383 mg Guanidin in 6 ml Methanol (abs.) über eine Zeitdauer von 45 Min. bei 60°. Nach Entfernung des Lösungsmittels und üblicher Aufarbeitung erhält man das N-Diaminomethylen-3-methylsulfonyl-4-(4-BOC-amino-piperidino)-benzamid, F. 224-226°.

### Beispiel 2

Man löst 3,4 g N-Diaminomethylen-3-methylsulfonyl-4-(4-BOC-aminopiperidino)-benzamid (F. 224-226°) in einer 2 n HCI-Lösung auf Dioxanbasis und rührt 1,5 Std. bei Raumtemperatur. Anschließend saugt man den kristallinen Rückstand ab und erhält nach Waschen mit Dioxan N-Diaminomethylen-3-methylsulfonyl-4-(4-amino-piperidino)-benzamid-Trihydrochlorid, F. 232-240°.

### Beispiel 3

3,9 g N-Diaminomethylen-3-methylsulfonyl-4-(4-amino-piperidino)-benzamid-Trihydrochlorid (F. 232-240°) werden in 50 ml Wasser gelöst und mit 1 n NaOH auf pH 12 eingestellt und gerührt. Der gebildete Niederschlag wird abgesaugt, mit 5 ml Wasser gewaschen und bei 50° getrocknet. Man erhält N-Diaminomethylen-3-methylsulfonyl-4-(4-amino-piperidino)-benzamid, F. 239-241°.

### Beispiel 4

2,5 g N-Diaminomethylen-3-methylsulfonyl-4-(4-amino-piperidino)-benzamid (F. 239-241°) werden in 75 ml Wasser suspendiert und unter Rühren mit 14,7 ml 1 n HCI versetzt. Nach Entfernung des Lösungsmittels und Lyophilisation erhält man N-Diaminomethylen-3-methylsulfonyl-4-(4-amino-piperidino)-benzamid-Dihydrochlorid, F. >260°.

### Beispiel 5

2,1 g N-Diaminomethylen-3-methylsulfonyl-4-fluor-benzamid [erhältlich durch Umsetzung von 3-Methylsulfonyl-4-fluor-benzoesäuremethylester mit Guanidin] werden mit 7,0 g 4-BOC-Amino-piperidin bei 150° zusammengeschmolzen. Nach 1,3 Std. Schmelzzeit läßt man die Mischung ab-kühlen und löst den Schmelzkuchen in 10 ml Dichlormethan/Methanol. Übliche Aufarbeitung und Chromatographie über Kieselgel (Ethylacetat/ Methanol) ergibt N-Diaminomethylen-3-methylsulfonyl-4-(4-BOC-aminopiperidino)-benzamid, F. 225-226°.

### Beispiel 6

1,0 g 3-Methylsulfonyl-4-(4-BOC-amino-piperidino)-benzoesäure [erhältlich durch Umsetzung von 3-Methylsulfonyl-4-fluor-benzoesäuremethylester mit 4-BOC-amino-piperidin und anschließende Verseifung des Produktes zur freien Säure] werden in 15 ml 1-Methylpyrrolidon gelöst, mit 0,67 g 1-Methyl-2-chlorpyridiniumchlorid versetzt und 15 Min. gerührt. Anschließend fügt man 0,9 g Guanidiniumchlorid sowie 2,6 ml Diisopropylethylamin hinzu und rührt 1 Std. bei Raumtemperatur. Nach üblicher Aufarbeitung erhält man nach Chromatographie über Kieselgel (Flash-Verfahren, Ethylacetat/10 % Methanol) N-Diaminomethylen-3-methylsulfonyl-4-(4-BOC-amino-piperidino)-benzamid.

### Beispiel 7

Analog Beispiel 6 erhält man durch Umsetzung von 3-Methylsulfonyl-4-(4-acetamido-piperidino)-benzoesäure mit Guanidiniumchlorid das N-Diaminomethylen-3-methylsulfonyl-4-(4-acetamido-piperidino)-benzamid-Hydrochlorid, F. 199-203°.

### Beispiel 8

Analog Beispiel 1 erhält man durch Umsetzung von 2-Methyl-4-(4-BOC-amino-piperidino)-5-methylsulfonyl-benzoesäuremethylester mit Guanidin das N-Diaminomethylen-2-methyl-4-(4-BOC-amino-piperidino)-5-methylsulfonyl-benzamid.

### Beispiel 9

Analog Beispiel 2 erhält man durch Entfernung der BOC-Schutzgruppen ausgehend
von N-Diaminomethylen-2-ethyl-4-(4-BOC-amino-piperidino)-5-methylsulfonyl-benzamid:
   N-Diaminomethylen-2-ethyl-4-(4-amino-piperidino)-5-methylsulfonylbenzamid;
von N-Diaminomethylen-2-trifluormethyl-4-(4-BOC-amino-piperidino)-5-methylsulfonyl-benzamid:
   N-Diaminomethylen-2-trifluormethyl-4-(4-amino-piperidino)-5-methylsulfonyl-benzamid;
von N-Diaminomethylen-2-chlor-4-(4-BOC-amino-piperidino)-5-methylsulfonyl-benzamid:
   N-Diaminomethylen-2-chlor-4-(4-amino-piperidino)-5-methylsulfonylbenzamid-Dihydrochlorid, F. 302-305°;
von N-Diaminomethylen-2-BOC-amino-4-(4-BOC-amino-piperidino)-5-methylsulfonyl-benzamid:
   N-Diaminomethylen-2-amino-4-(4-amino-piperidino)-5-methylsulfonylbenzamid;
von N-Diaminomethylen-3-trifluormethyl-4-(4-BOC-amino-piperidino)-benzamid:
   N-Diaminomethylen-3-trifluormethyl-4-(4-amino-piperidino)-benzamid-Trihydrochlorid, F. 235°;
von N-Diaminomethylen-2-methyl-4-(4-BOC-amino-piperidino)-5-methylsulfonyl-benzamid:
   N-Diaminomethylen-2-methyl-4-(4-amino-piperidino)-5-methylsulfonyl-benzamid-Dihydrochlorid, F. 305-310°;
von N-Diaminomethylen-2-cyan-4-(4-BOC-amino-piperidino)-5-methylsulfonyl-benzamid:
   N-Diaminomethylen-2-cyan-4-(4-amino-piperidino)-5-methylsulfonylbenzamid;
von N-Diaminomethylen-2-hydroxy-4-(4-BOC-amino-piperidino)-5-methylsulfonyl-benzamid:
   N-Diaminomethylen-2-hydroxy-4-(4-amino-piperidino)-5-methylsulfonyl-benzamid;
von N-Diaminomethylen-2-methoxy-4-(4-BOC-amino-piperidino)-5-methylsulfonyl-benzamid:
   N-Diaminomethylen-2-methoxy-4-(4-amino-piperidino)-5-methylsulfonyl-benzamid-Dihydrochlorid, F. 270°;
von N-Diaminomethylen-3-methylsulfonyl-4-(4-BOC-amino-piperidino)-5-nitro-benzamid:
   N-Diaminomethylen-3-methylsulfonyl-4-(4-amino-piperidino)-5-nitrobenzamid-Dihydrochlorid, F. 264°.

Die nachstehenden Beispiele betreffen pharmazeutische Zubereitungen.

### Beispiel A: Injektionsgläser

Eine Lösung von 100 g einer erfindungsgemäßen Verbindung nach Anspruch 1 und 5 g Dinatriumhydrogenphosphat werden in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g einer erfindungsgemäßen Verbindung nach Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g einer erfindungsgemäßen Verbindung nach Anspruch 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann beispielsweise in Form von Augentropfen verwendet werden.

### Beispiel D: Salbe

Man mischt 500 mg einer erfindungsgemäßen Verbindung nach Anspruch 1 mit 99,5 g Vaseline unter aseptischen Bedingungen.

### Beispiel E: Tabletten

Ein Gemisch von 1 kg einer erfindungsgemäßen Verbindung nach Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg einer erfindungsgemäßen Verbindung nach Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg einer erfindungsgemäßen Verbindung nach Anspruch 1 in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. N-Diaminomethylen-4-(4-amino-piperidino)-5-methylsulfonyl-benzamidderivate
a) N-Diaminomethylen-4-(4-amino-piperidino)-2-methyl-5-methylsulfonyl-benzamid,
b) N-Diaminomethylen-4-(4-amino-piperidino)-2-methoxy-5-methylsulfonyl-benzamid,
c) N-Diaminomethylen-4-(4-amino-piperidino)-2-chlor-5-methylsulfonyl-benzamid,
sowie deren physiologisch unbedenklichen Salze.

2. Verfahren zur Herstellung pharmazeutischer Zubereitungen, **dadurch gekennzeichnet**, daß man eine Verbindung nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

3. Pharmazeutische Zubereitung, gekennzeichnet durch einen Gehalt an mindestens einer Verbindung nach Anspruch 1 und/oder einem ihrer physiologisch unbedenklichen Salze.

4. Verwendung von Verbindungen nach Anspruch 1 oder von deren physiologisch unbedenklichen Salzen zur Herstellung eines Arzneimittels.

5. Verwendung von Verbindungen nach Anspruch 1 und/oder ihre physiologisch unbedenklichen Salze zur Herstellung von Arzneimitteln zur Behandlung von Arrhythmien, Angina pectoris, Infarkten sowie zur präventiven Behandlung der genannten Indikationen.

## Claims

1. N-Diaminomethylene-4-(4-aminopiperidino)-5-methylsulfonylbenzamide derivatives
a) N-diaminomethylene-4-(4-aminopiperidino)-2-methyl-5-methylsulfonylbenzamide,
b) N-diaminomethylene-4-(4-aminopiperidino)-2-methoxy-5-methylsulfonylbenzamide,
c) N-diaminomethylene-4-(4-aminopiperidino)-2-chloro-5-methylsulfonylbenzamide,
and the physiologically harmless salts thereof.

2. Process for producing pharmaceutical preparations, **characterized in that** a compound according to Claim 1 and/or one of its physiologically harmless salts is/are brought into a suitable dosage form together with at least one solid, liquid or semiliquid excipient or adjuvant.

3. Pharmaceutical preparation, characterized by a content of at least one compound according to Claim 1, and/or one of its physiologically harmless salts.

4. Use of compounds according to Claim 1 or of their physiologically harmless salts for producing a drug.

5. Use of compounds according to Claim 1 and/or their physiologically harmless salts for producing drugs for the treatment of arrhythmias, angina pectoris and infarctions and also for the preventive treatment of the said indications.

## Revendications

1. Les dérivés de N-diaminométhylène-4-(4-aminopipéridino)-5-méthylsulfonylbenzamide
a) le N-diaminométhylène-4-(4-aminopipéridino)-2-méthyl-5-méthylsulfonylbenzamide,
b) le N-diaminométhylène-4-(4-aminopipéridino)-2-méthoxy-5-méthylsulfonylbenzamide,
c) le N-diaminométhylène-4-(4-aminopipéridino)-2-chloro-5-méthylsulfonylbenzamide,
ainsi que leurs sels physiologiquement acceptables.

2. Procédé pour la fabrication de préparations pharmaceutiques, **caractérisé en ce que** l'on met sous une forme de dosage appropriée un composé selon la revendication 1 et/ou l'un de ses sels physiologiquement acceptables, associé à au moins un support ou un adjuvant solide, liquide ou semi-liquide.

3. Préparation pharmaceutique **caractérisée en ce qu**'elle contient au moins un composé selon la revendication 1 et /ou l'un de ses sels physiologiquement acceptables.

4. Utilisation des composés selon la revendication 1 ou de leurs sels physiologiquement acceptables pour la fabrication d'un médicament.

5. Utilisation des composés selon la revendication 1 et/ou de leurs sels physiologiquement acceptables pour la fabrication de médicaments destinés au traitement des arythmies, de l'angine de poitrine, des infarctus, ainsi qu'au traitement préventif des indications citées.
